(19) Europäisches Patentamt / European Patent Office / Office européen des brevets

(11) **EP 4 623 919 A1**

(12) **EUROPEAN PATENT APPLICATION**

(43) Date of publication:
01.10.2025  Bulletin 2025/40

(21) Application number: 24167732.7

(22) Date of filing: 28.03.2024

(51) International Patent Classification (IPC):
**A61K 31/731** (2006.01)     **A61K 9/00** (2006.01)
**A61P 27/04** (2006.01)     **A61P 27/06** (2006.01)
**A61P 27/14** (2006.01)

(52) Cooperative Patent Classification (CPC):
**A61K 31/731; A61K 9/0048; A61P 27/04;
A61P 27/06; A61P 27/14**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Marinomed Biotech AG
2100 Korneuburg (AT)**

(72) Inventors:
• **PRIESCHL-GRASSAUER, Eva
1210 Wien (AT)**

• **Roch-Nakowitsch, Sabine
3443 Sieghartskirchen, (AT)**
• **MOROKUTTI-KURZ, Martina
1130 Wien (AT)**
• **Russo, Antonella
3400 Klosterneuburg, (AT)**
• **KOLLER, Christiane
2201 Seyring/Gerasdorf (AT)**

(74) Representative: **Bogensberger, Burkhard
Bogensberger Patent- & Markenbüro
Widagass 47
9487 Gamprin-Bendern (LI)**

(54) **COMPOSITION COMPRISING IOTA-CARRAGEENAN FOR THE TREATMENT OF DRY EYES, NON-VIRAL CONJUNCTIVITIS AND ALLERGIC OCULAR CONDITIONS**

(57) The present invetion relates to iota-carrageenan for use as an active ingredient in an aqueous pharmaceutical composition for the prevention or treatment of clinically relevant eye conditions affecting at least one of mucosal tissues at or around an individual's eye, an eye surface, and corneal tissue, said clinically relevant eye conditions selected from the group consisting of dry eye, non-viral conjunctivitis, irritations caused by airborne particulate matter and allergens, and increased ocular pressure.

FIG.1A

EP 4 623 919 A1

**Description**

TECHNICAL FIELD

The present invention relates to a pharmaceutical composition comprising iota-carrageenan for use as a medicament in the prophylactic and/or therapeutic treatment of dry eyes and related conditions in human individuals and non-human animals.

BACKGROUND OF THE INVENTION

In healthy individuals, a protective film of tears coats the eye's outer layer. The right amount and quality of tears keep the eyes nourished and lubricated. With every blink, a protective film of basal tears spreads over the surface of one's eye in order to provide lubrication for reducing friction from blinking, but also for washing away unwanted substances and particles from the eyes and for keeping the surface of the eyes clear.

Tears have three layers - the outer lipid layer, the middle aqueous layer, and the inner mucin layer. The inner layer is made up of mucus that coats the surface of the eye. The mucin layer binds tears to the eye. More specifically, since the cornea is hydrophobic in nature the mucous layer helps to connect the adjacent watery layer and to spread it evenly over the surface of the eye.

The watery layer, which is the middle layer, is more like a saline solution and contains vitamins and minerals that keep the eye nourished. It is also responsible for maintaining the eye lubricated and for washing away unwanted particles and substances.

The outermost layer of the tear film is an oil or lipid-based layer. It keeps the surface of the tear film smooth and prevents the watery layer from drying up too quickly.

Dry eyes can occur when there is a loss of natural balance with any of these layers. Dry eye may result from a variety of causes, among which aging being one of the most prominent causes. As people age, the production of tears decreases affecting both women and men and especially post-menopausal women.

Other causes of dry eye include certain illnesses such as rheumatoid arthritis, lupus, Graves' disease, diabetes, scleroderma, and Sjogren's syndrome; but also hormonal changes in women after menopause and during pregnancy.

Typically, poor blinking while reading or looking at a computer screen for long periods of time may also cause dry eyes, as well as exposure to a dry indoor or outdoor environment combined with constant or repeated airflow stress to the eyes, as caused, for example, by air-conditioning systems and windy outdoor conditions. The use of contact lenses may also lead to dry eyes. And last but not least unhealthy nutrition, but also various medications including tranquilizers, antihistamines, certain heart medications, diuretics, birth control pills, and ulcer medications may contribute to the development of dry eyes and related symptoms and conditions.

Frequently, dry eye symptoms include a dry, gritty or burning sensation in the eyes, redness, watery or teary eyes and mucus. Many people also report the feeling of something in the eye or eyestrain. Itching and light sensitivity may also occur.

If dry eye is left untreated, it can damage eye tissue and mucous membranes around the eyes and also cause serious injuries to the cornea. Prophylactic and/or therapeutic treatment of dry eye is typically directed at wetting the eye, and possibly reducing inflammation, in addition to further measures directed at avoiding the afore-mentioned environmental, nutritional, medication-related, and other stress factors usually involved with the development of dry eye symptoms and conditions.

Various polymers, especially polysaccharides, are known to have water-binding and moisturizing properties and can help alleviate discomfort by providing lubrication and wetting to affected membranes and tissues.

Apart from or in addition to above-mentioned internal and external triggers of dry eye symptoms there are further factors causing discomfort to the eye. Especially the presence of airborne particulate matter (PM) including fine dust particles, pollen of various origins, and other small particles typically carried by outdoor winds, can adversely affect eye functionality and vision.

Various polymers, among which hydroxypropylmethyl cellulose (HPMC), are known as viscosity enhancers having additional functionality as builders of physical or physico-chemical barriers against airborne intruders such as viruses, bacteria, allergens, and small particulate matter, at mucosal membranes or tissues of the nose, mouth and the eyes, i.e. at the usual site of entry of such airborne stuff.

The use of carrageenans as excipients and viscosifiers in ophthalmology is well established. United States patent no. 5,403,841 describes carrageenan-based solutions that are useful for preparing eye drop formulations of pharmaceutically active ingredients. On contact with the tear film the solutions form a gel which maintains extended contact with the conjunctiva, preventing quick removal of the active principle from the eye surface and facilitating its topical delivery.

Some ocular pharmaceutical preparations that employ carrageenans as excipients and/or mucomimetics contain antiviral agents. For example, international patent application WO 2007/039201 claims photo-stable formulations of brivudine to treat herpetic keratitis.

Ophthalmic preparations based on natural organic polymers are known to have been designed explicitly for treating conjunctival inflammation. For example, international patent application WO 2005/046562 claims sulphated hyaluronic acids for such a therapeutic purpose.

BRIEF DESCRIPTION OF THE INVENTION

In order to find an improved alternative for the prophylactic and/or therapeutic treatment of dry eye symptoms and for the prevention of eye irritations caused by small particulate matter, especially airborne particulate matter such as particles including fine dust particles, pollen of various origin, allergens, animal dander, and other small particles typically carried by air-conditioning systems or outdoor winds, a selection of known polymers was tested for suitability as possible candidates for the manufacture of eye drops useful in the prevention or treatment of dry eye symptoms and related conditions. The polymers were tested and compared with each other with respect to the following properties and capacities:

- adhesion strength and duration of adhesion to mucosal surfaces;
- wetting capacity and capacity of preventing early drying out of the tear film;
- protective efficacy for corneal tissue and cells, i.e. efficacy of maintaining corneal tissue and cells vital over an observation period under adverse circumstances;
- barrier function against penetration by small particulate matter, frequently particulate matter in the micrometer range.

The tested polymers were selected from the group of high molecular weight iota-carrageenan (about 3 - 10 MDa), kappa-carrageenan (about 5-10 MDa), lambda-carrageenan (about 5-10 MDa), carboxymethyl cellulose (CMC) of high viscosity, hydroxypropylmethyl cellulose (HPMC), fucoidan 1 from the algae *undaria pinnitafida,* fucoidan 2 from the brown algae *fucus vesiculosus,* dextran sulfate, and low molecular weight (i.e. chemically degraded) iota-carrageenan (about 100 - 500 kDa). The average molecular weight of the carrageenan fractions was determined by size exclusion HPLC in combination with refraction index measurement and evaluated against dextrans with 25, 50, 150, 410, 670 and 1100 kDa as standards.

The results of the experimental comparative studies revealed a remarkable and unexpected superiority of iota-carrageenan in comparison to all tested polymers. Iota-carrageenan demonstrated consistent and effective moisturizing properties as well as a strong activity in protecting the ocular surface against drying out. In addition, it proved most effective in blocking particulate matter from penetrating a polymer barrier layer.

It is therefore an objective of the present invention to provide an alternative treatment for the prophylaxis or therapy of dry eye symptoms, and for the protection of the cornea and of mucosal tissues around the eyes of a mammalian, especially human, individual against intrusion and possible damage by small particulate matter including dust, pollen, and allergens. As a consequence, the present invention is helpful in the prevention and treatment of non-viral induced clinically relevant symptoms and conditions of the outer eye including non-viral induced conjunctivitis.

DETAILED DESCRIPTION OF THE INVENTION

In the context of the present invention, the terms "therapy" or "therapeutical treatment" shall refer to medical interventions that are intended to modify the clinical course of dry eye symptoms or of a non-viral conjunctivitis in such a way that either at least one of the clinical symptoms such as ocular redness, pain, and disturbances of vision and tear flow, are less severe than without such interventions; or that said clinical symptoms persist for shorter periods, i.e. resolve earlier.

Likewise, in the context of the present invention, the terms "prophylaxis", "prophylactic treatment" or "prevention" shall mean that pretreatment of a healthy eye in line with the present invention reduces the likelihood of developing clinically relevant symptoms of eye irritation or dry eye disease including non-viral conjunctivitis. It shall also be understood as reducing the severity and/or duration of clinically relevant symptoms of eye irritation and dry eye disease including non-viral conjunctivitis, if pretreatment of the healthy eye occurs to prior to an individual's exposure to circumstances that absent such pretreatment would likely cause eye irritations, dry eye symptoms, or a non-viral conjunctivitis.

The present invention is also helpful in preventing or ameliorating late complications of non-viral conjunctivitis. Such complications are known in the scientific and clinical literature, and include - but are not limited to - corneal opacities, subepithelial infiltrates, and formation of ocular pseudo membranes.

The preparations according to the present invention typically contain carrageenan, preferably iota-carrageenan, as the main active moisturizing, protective and soothing ingredient or as the sole active moisturizing, protective and soothing ingredient at a concentration of from 0.05% to 1% by weight, preferably of from 0.1 to 0.5%, and most preferably of from 0.1 to 0.35 % by weight of the ready-for-use preparation. Also, preparations in line with the invention are preferably free from sodium or potassium chloride, or contain no more than 0.5% (w/v), preferably no more than 0.1% (w/v) of one or these salts.

Topically administrable ophthalmic compositions according to the present invention have a pH value within a range of from 3.5 to 8.5, usually a pH value in the range of from about 5.5 to about 8.5, and an osmolality of about 220 to 340 mOsm/kg. However, for various applications it may be preferable to adjust the osmolality to slightly hypotonic values, said values typically being within a range of from 170 to 250 mOsm/kg, and more specifically within a range of from 190 - 220 mOsm/kg, in order to compensate for hypertonicity of the tear film due to disease or excessive evaporation with patients suffering from non-viral conjunctivitis.

According to the present invention adjustment of osmolality is accomplished without the use of metal halide salts such as NaCl or KCl. Instead, the desired osmolality may be adjusted by adding at least one of a low molecular weight sugar and a low molecular weight polyvalent alcohol ("polyol"). Suitable sugars may be selected from the group of monosaccharides, disaccharides, and oligosaccharides, and typically from glucose, fructose, mannose, and sucrose. Suitable polyvalent alcohols, typically short-chain sugar alcohols having a backbone of 3 to 12 carbon atoms, may be selected from the group of glycerol, erythritol, sorbitol, mannitol, xylitol, threitol, inositol, and maltitol.

The ophthalmic compositions according to the present invention may comprise one or more ophthalmologically compatible pH adjusting agents or buffer systems that prevent pH drift during storage. Such agents include, but are not limited to, boric acid, sodium borate, potassium citrate, citric acid, sodium bicarbonate, and various inorganic phosphate buffers such as $Na_2HPO_4$, $NaH_2PO_4$, $KH_2PO_4$, and mixtures thereof.

Also, the present ophthalmic compositions may comprise one or more ophthalmologically compatible surfactants. The surfactant facilitates the spread of the liquid aqueous solution across the surface of the eye, and may be non-ionic or anionic. Exemplary non-ionic surfactants include tyloxapol, polyoxyethylene sorbitan esters, polyethoxylated castor oils, poloxamers, polyoxyethylene/polyoxypropylene surfactants, polyoxyethylene stearate, polyoxyethylene propylene glycol stearate, hydroxyalkylphosphonate, lauric or palmitic acid esters and ethers, triethanol amine oleate, or a combination of the foregoing agents, or other agents known to those skilled in the art. The surfactant when included is typically present at a concentration of between 0.02 % (w/v) and 0.1 % (w/v) of the composition.

The present ophthalmic compositions may also contain one or more preservatives to inhibit microbial growth and to prolong shelf life. Exemplary preservatives include, but are not limited to, benzalkonium chloride, polyquaternium-1, polyhexamethylene biguanide, and perborate. The preservative amount is typically about 0.01 % (w/v) or less of the total composition.

Also, disodium edetate (EDTA) may be present in the present compositions in line with the invention, typically at a concentration within a range of from 0.05 to 2 mg/ml (0.005 - 0.2% w/v).

In addition to the ingredients above, it is contemplated that a variety of additional or alternative ingredients may be present in the ophthalmic pharmaceutical compositions of the present invention, which alternative ingredients include without limitation anti-oxidants such as vitamin E or its commercially available derivatives such as tocopherol polyethylene glycol 1000 succinate (TPGS), ascorbic acid, or sodium metabisulfite.

The pharmaceutical compositions for ophthalmic use according to the present invention are typically provided in sterile form suitable for topical administration to the frontal part of the eye, and are preferably adjusted for self-administration by the individual in need thereof. In one embodiment, the composition is a particle-free eye drop solution. Various containers are known in the art that are suitable for dispensing of liquids drop by drop to the ocular surface through a nozzle in a fashion that can be easily controlled by an individual during self-administration of said drops. Preferably, a typical container+ nozzle system is designed to maintain sterility of the eye drops during repeated use.

Other galenic formulations within the scope of the invention include ophthalmologically acceptable swabs, ointments, or gels that can be applied to the eye as sprays or aerosols, or gels that can be administered into the conjunctival sac. For each of these formulations various products or application systems are known in the art that are designed to dispense such formulations to the front of the eye without risking mechanical damage to the ocular surface.

For special applications the present pharmaceutical compositions may also be formulated into controlled release devices that are either transiently placed into the conjunctival sac, or dissolve in situ while they release the iota-carrageenan preparation according to the invention.

BRIEF DESCRIPTION OF THE DRAWINGS

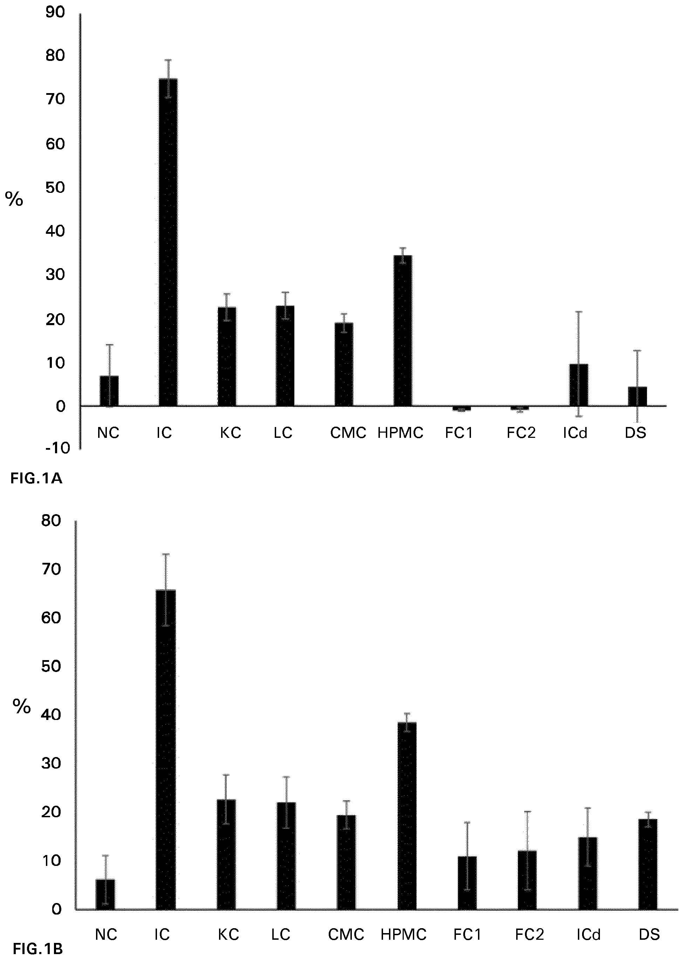

Fig.1A, 1B: graphical representation of cell-protective efficacy of selected polymers, expressed as % cell survival after 10 minutes exposure to dehydrating conditions, relative to non-dehydrated control cells; error bars represent the standard deviation; y-axis: % of viable cells relative to control; x-axis: selected polymers tested: NC = negative control (unprotected cells), IC = iota carrageenan, KC = kappa-carrageenan, LC = lambda-carrageenan, CMC = carboxymethyl cellulose of high viscosity, HPMC = hydroxypropylmethyl cellulose, FC1 = fucoidan from undaria pinnatifida, FC2 = fucoidan from fucus vesiculosus, ICd = degraded, low molecular weight iota-carrageenan, DS=dextran sulphate; Fig. 1A: results on human cornea cells; Fig. 1B: results on human lung cells.

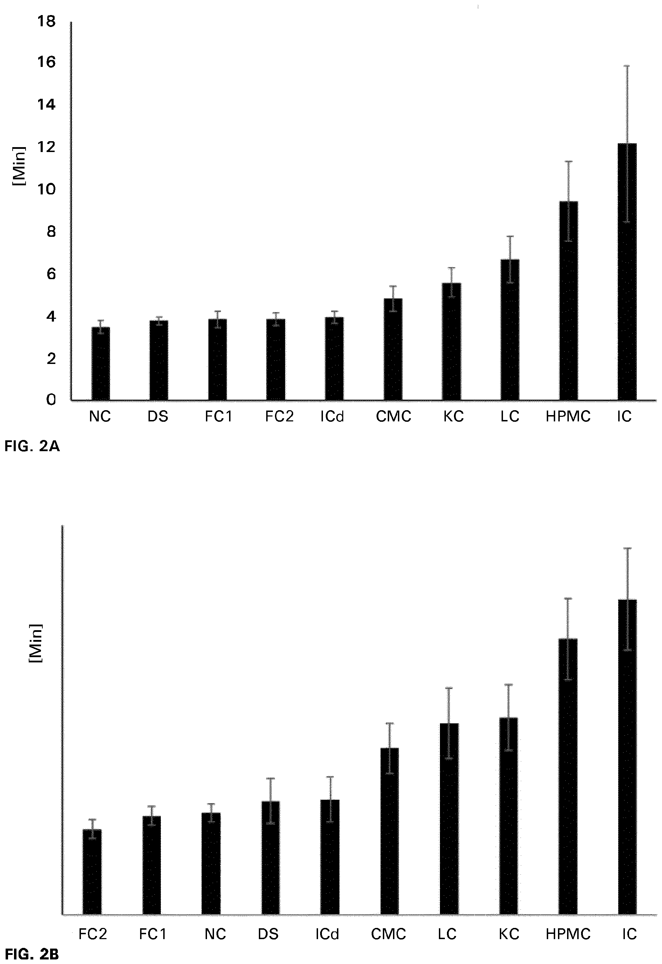

Fig. 2A, 2B: graphical representation of cell-protective efficacy of selected polymers, expressed as half-life of cells pre-treated with polymer and thereafter exposed to dehydrating conditions of up to 40 minutes; error bars represent the standard deviation; y-axis: time in minutes during which 50% of the cells remain viable ( = half-life); x-axis: selected polymers tested: NC = negative control (unprotected cells), IC = iota carrageenan, KC = kappa-carrageenan, LC = lambda-carrageenan, CMC = carboxymethyl cellulose of high viscosity, HPMC = hydroxypropylmethyl cellulose, FC1 = fucoidan from undaria pinnatifida, FC2 = fucoidan from fucus vesiculosus, ICd = degraded, low molecular weight iota-carrageenan, DS = dextran sulphate; Fig. 2A: results on human cornea cells; Fig. 2B: results on human lung cells.

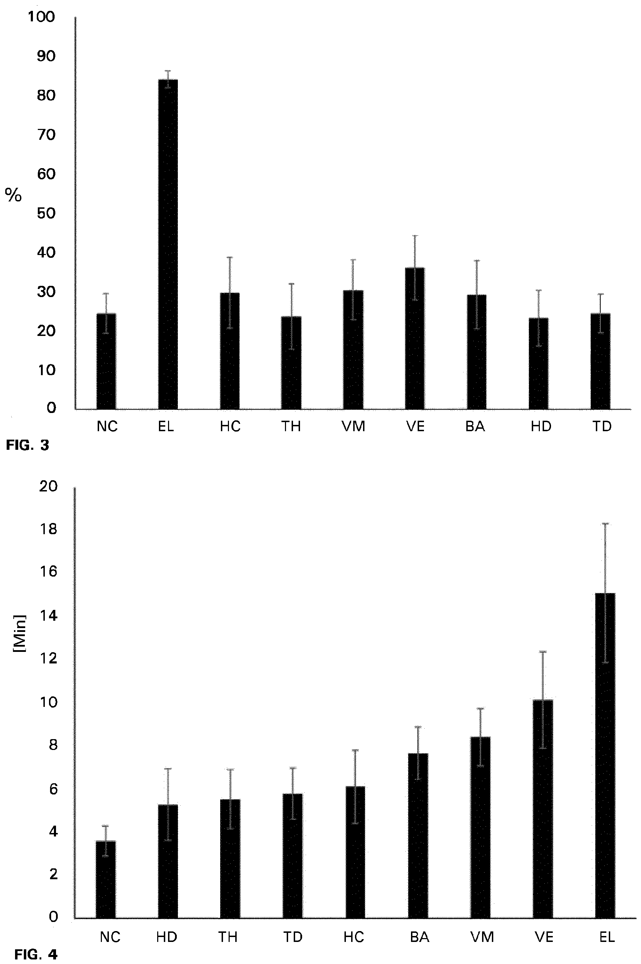

Fig. 3: graphical representation of cell-protective efficacy of selected commercial eye-drop solutions, expressed as % cell survival of pre-treated corneal cells after 10 minutes exposure to dehydrating conditions, relative to non-dehydrated control cells; error bars represent the standard deviation; y-axis: % of viable cells relative to control; x-axis: commercial preparations tested: NC = negative control (unprotected cells), EL comprises 0.32% w/v iota-carrageenan, HC comprises 0.10 % HA, TH comprises 0.15% HA, VM comprises 0.18% HA, VE comprises 0.24% HA + 2% ectoin, BA comprises 0.15% HA, HD comprises 0.05% HA + 2% ectoin, TD comprises 0.15% HA + 3% trehalose;

Fig. 4: graphical representation of cell-protective efficacy of selected commercial eye-drop solutions, expressed as half-life of corneal cells pre-treated with commercial eye-drop preparation and thereafter exposed to dehydrating conditions of up to 40 minutes; error bars represent the standard deviation; y-axis: time in minutes during which 50% of the cells remain viable (= half-life); x-axis: commercial preparations tested: NC = negative control (unprotected cells), EL comprises 0.32% w/v iota-carrageenan, HC comprises 0.10 % HA, TH comprises 0.15% HA, VM comprises 0.18% HA, VE comprises 0.24% HA + 2% ectoin, BA comprises 0.15% HA, HD comprises 0.05% HA + 2% ectoin, TD comprises 0.15% HA + 3% trehalose;

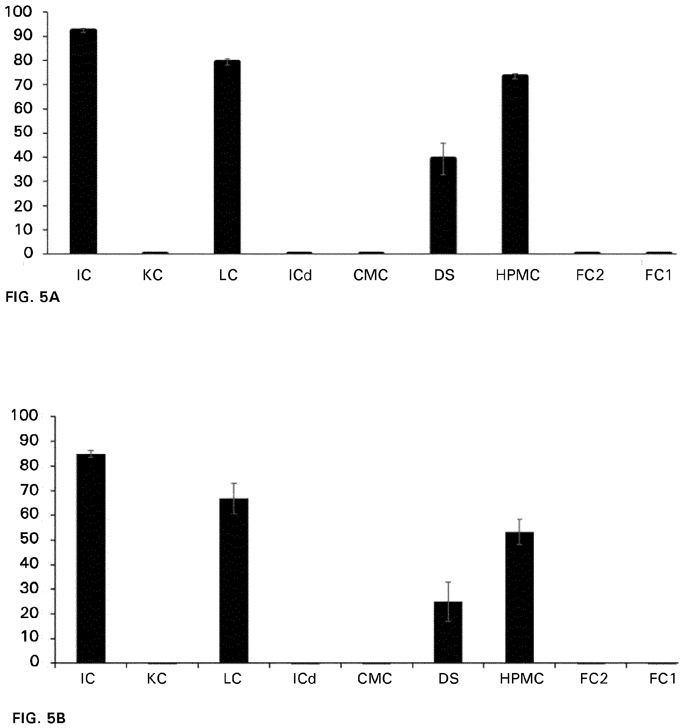

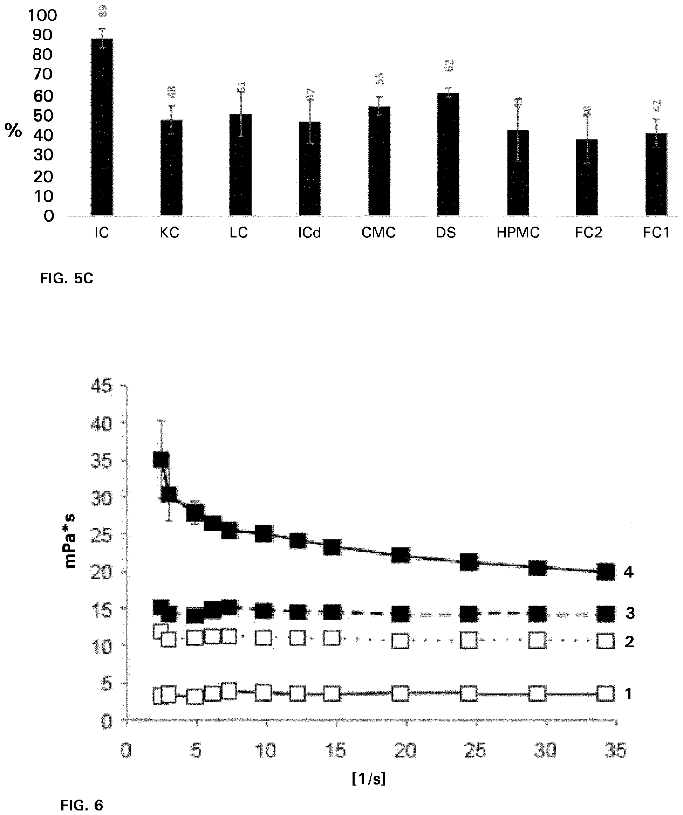

Fig. 5A, 5B, 5C: graphical representation of the effectiveness of different polymers to form a barrier layer for blocking the migration of particulate matter through the barrier layer in an in vitro assay, expressed as percentage of blocked particulate matter relative to total applied amount of particulate matter; y-axis: % of blocked particulate matter (% blocking efficacy), x-axis: polymers tested for blocking efficacy; IC = iota carrageenan, KC = kappa-carrageenan, LC = lambda-carrageenan, CMC = carboxymethyl cellulose of high viscosity, HPMC = hydroxypropylmethyl cellulose, FC1 = fucoidan from undaria pinnatifida, FC2 = fucoidan from fucus vesiculosus, ICd = degraded, low molecular weight iota-carrageenan, DS = dextran sulphate; Fig. 5A: results obtained with 0.3 $\mu$m fluorescent beads, Fig. 5B: results obtained with 1 $\mu$m fluorescent beads, Fig. 5C: results obtained with Der p 1 (house dust mite) allergen.

Fig. 6: graphical representation of viscosity measurements at shear rates of from 2.44 to 34.2 [1/s] on a Searle type viscosimeter for determination of the mucoadhesive properties of a iota-carrageenan eye-drop solution; y-axis: viscosity in [mPa*s]; x-axis: shear rate in [1/s]; 1 = iota-carrageenan preparation comprising 2.13 mg/ml iota-carrageenan; 2= 5% mucin solution; 3= calculated additive value of viscosities of 1 and 2; 4= measured viscosity of a mixture of 1 (iota-carrageenan) and 2 (mucin); data represent the mean of triplicate measurements; standard deviation is indicated.

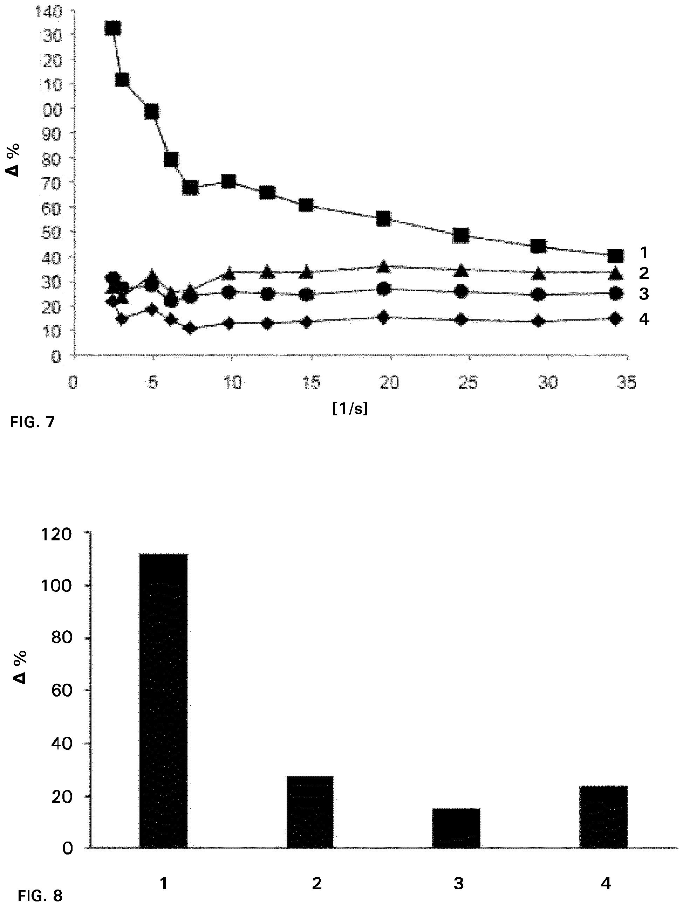

Fig. 7: graphical representation of a comparison of mucoadhesive strength determined at shear rates of from 2.44 to 34.2 [1/s];
y-axis: mucoadhesion expressed in % of viscosity increase over the calculated additive value of independently determined viscosities of the respective polymer and mucin solutions; x-axis: applied shear rates in [1/s]; 1 = iota-carrageenan + mucin, 2= commercial HD + mucin; 3= commercial SY + mucin, 4= commercial TD + mucin.

Fig. 8: graphical representation of a comparison of mucoadhesive strength determined at a shear rate of 3 [1/s]; y-axis: mucoadhesion expressed in % of viscosity increase over the calculated additive value of independently determined viscosities of the respective polymer and mucin solutions; x-axis: experimental samples of iota-carrageenan and commercial eye-drop preparations in admixture with 5% mucin; 1 = iota-carrageenan 2.13 mg/ml + 5% mucin; 2 = commercial SY + 5% mucin; 3= commercial TD + 5% mucin, 4= commercial HD + 5% mucin.

EXAMPLE 1: Determination of moisturizing and eye protective capacity of selected polymers

The search of effective eye care products has led to the development of numerous eye drop formulations promising relief from dryness and discomfort. Among these, assessing the moisturizing efficacy and protection capacity against drying out of the eye surface becomes vital to ensure optimal ocular health and comfort.

In a first experiment, selected polymers were tested and compared for moisturizing and eye protective capacity.

Abbreviations used:

| | |
|---|---|
| CEpiGF | Growth supplements |
| CCM | Cell culture medium |
| CMC | Carboxymethyl cellulose |
| DMEM | Dulbecco's Modified Eagle's Medium |
| FBS | Fetal bovine serum |
| huCEpiC | Human immortalized Cornea Cells |
| HPMC | Hydroxypropylmethyl cellulose |
| IM-OEpiCM | IM-Ocular Epithelial Cell Medium |
| iPC | Internal positive control |
| RT | Room Temperature |
| sPC | Sample positive control |
| NC | Negative control |

The polymers were each provided in cell culture medium at a concentration of 1.2 mg/ml.

Table 1: Identification and origin of applied polymers

| Substance and quality (MAM number) | Supplier |
|---|---|
| Iota-carrageenan (MAM-1001) Gelcarin PH379 | Du Pont Nutrition |
| Kappa911-carrageenan (MAM-1002) Gelcarin PH911 | Du Pont Nutrition |
| Lambda-carrageenan Viscarin PH109 | FMC |
| Dextran sulphate sodium salt from Leuconostoc spp., average mol wt > 500,000 (dextran starting material), contains 0.5-2.0% phosphate buffer, pH 6-8 | Sigma |
| Fucoidan from Undaria pinnatifida > = 95% | Sigma |
| Fucoidan from Fucus vesiculosus > = 95% | Sigma |
| CMC high viscosity carboxymethyl cellulose | Hercules |
| HPMC hydroxypropylmethyl cellulose E4M PREM | Fagron |
| Degraded low-molecular weight iota-carrageenan | in-house |

Table 2: Cells used for comparative viability study

| Cells | Passage | Cultivation medium |
|---|---|---|
| Human immortalized Cornea Cells (huCEpiC) | p8 | IM-OEpiCM + 5 % FBS + 1 % P/S + 1 % CEpiGF |
| A549 (human lung cells) | p8 | DEMEM + 10 % FBS |

The day before the onset of the experiment huCEpi cells as well as A549 cells were seeded into 96-well plates. Four wells of each plate were left empty serving as blank controls for the analysis of the experiments. The following controls were established:

iPC: is the internal control where cells were treated throughout the experiment with cell culture medium and were not subjected to dehydrating conditions.

sPC: is the sample positive control where cells were treated with a mixture of one part sample medium and one part cell culture medium throughout the experiment and were not subjected to dehydrating conditions.

NC: is the negative control where cells were pre-treated solely with cell culture medium and subjected to drying conditions thereafter.

The following day the seeding medium was replaced with the polymer samples in CCM. For the iPC and NC the medium was replaced with cell culture medium only, i.e. not containing any polymer. The cells were incubated under cell culture conditions for 2 h. Treated and non-treated (NC) cells were then subjected to dehydrating conditions.

The terms "dehydration" and "dehydrating conditions" as used herein shall be understood as referring to conditions that cause evaporation of moisture from a watery or humid surface such as the ocular surface, or from a liquid aqueous sample surface in an in vitro setting.

The dehydrating conditions applied to the experimental cell cultures comprised removing the medium and placing the plates in a dry, moisture-free incubator at 33 °C. For determining the capacity of the different polymers in keeping 50 % of the cells viable a dehydration time course was applied from 2 up to 25 minutes. The iPC and sPC were not subjected to these dehydrating conditions. The cell viability was then evaluated using Alamar blue containing resazurin, wherein in viable, i.e. metabolically active cells, resazurin is reduced to resorufin by mitochondrial enzymes, which change can be detected using optical and imaging methods.

Cell viability was calculated relative to the corresponding sPC (= 100% viability). Each sample was tested in triplicates. For analyzing the half-life survival of cells after treatment with the experimental samples, the time point was calculated when 50 % of the metabolic signal was still detectable.

Results

The blank values were subtracted from all samples. For each polymer preparation the survival of cells in % of the corresponding positive control was calculated. Moreover, with the use of the Excel plug-in XLfit a dose response analysis was performed calculating the time in which 50% of the cells are still viable after exposure to the dehydrating conditions.

The bar charts of Figures 1A and 1B exhibit the results of cell protection by the tested polymers as percentage of cell survival relative to the corresponding sPC, determined after dehydrating the sample preparations for 10 minutes under the conditions mentioned above. Fig.1A refers to the results obtained with human corneal cells, Fig. 1B to those obtained with A549 lung cells.

The bar charts in Figs. 2A and 2B exhibit a comparison of the cell protective efficacy of the individual polymer preparations, expressed as the average time during which 50% of the corneal cells (Fig. 2A) and of the lung cells (Fig.2B) were kept alive, i.e. metabolically active, after dehydrating treatment for up to 40 minutes.

It shall be mentioned at this occasion that the commercially available "lambda-carrageenan" material typically contains only relatively low amounts of lambda carrageenan and much higher amounts of kappa- and iota-carrageenan. The composition of the lambda-carrageenan material used in the present experiments comprised 41.8 % wt of kappa-carrageenan, 27.3 %wt of iota-carrageenan, and only 19.6 % wt of lambda-carrageenan, as determined using a validated NMR detection method.

The results revealed a notable superiority of iota-carrageenan in wetting cell protective capacity compared to all other polymers tested. The observed half-life of human cornea cells as well as of human lung cells (A549) subjected to preceding treatment with iota-carrageenan prior to the dehydration run is approximately 12 minutes, indicating a remarkable enhancement in cell viability of approximately 3.5-fold as compared to the unprotected negative control. In contrast, treatment with all other tested polymers resulted in half-lives significantly shorter than with iota-carrageenan, indicating less effective moisturizing properties.

EXAMPLE 2: Comparison of moisturizing and eye protective efficacy of some commercial eye-drop preparations with the present iota-carrageenan based composition.

The goal of this experiment was to compare the efficacy of different commercially available eye-drop products with respect to preventing evaporation of moisture from the ocular surface and keeping the cornea cells humid and vital. Each one of the commercial products contained a known moisturizing agent, i.e. hyaluronic acid (HA), ectoin, or trehalose.

For this purpose, the experimental set up of EXAMPLE 1 was applied again.

The present iota-carrageenan composition EL, i.e. Sample 1, comprised 0.32% w/v iota-carrageenan. This composition was tested against commercial products HC (Sample 2) comprising 0.10% hyaluronic acid (HA), TH (Sample 3) comprising 0.15% HA, VM (Sample 4) comprising 0.15% HA, VE (Sample 5) comprising 0.24% HA plus 2% ectoin, BA (Sample 6) comprising 0.15% HA, HD (Sample 7) comprising 0.05% HA plus 2% ectoin, and TD (Sample 8) comprising 0.15% HA plus 3% trehalose (see Table 3). The in vitro test was designed to evaluate the protective efficacy against dehydration of cornea cells under adverse drying conditions.

Table 3: Commercial products used and active ingredients contained therein

| Sample | Active ingredient |
|---|---|
| 1 /EL | 0.32 % iota-carrageenan |
| 2 /HC | 0.10 % HA |
| 3 /TH | 0.15 % HA |
| 4 /VM | 0.18 % HA |
| 5 /VE | 0.24 % HA; 2% ectoin |
| 6 /BA | 0.15 % HA |
| 7 /HD | 0.05 % HA; 2% ectoin |
| 8 /TD | 0.15 % HA; 3% trehalose |

All of the above samples were diluted with cell culture medium to yield 50 % of the original concentration, before they were subjected to the experimental conditions.

As disclosed in EXAMPLE 1, huCEpi cells were seeded into 96-well plates the day before the onset of the experiment. The last four wells of each plate were left empty serving as blank controls for the analysis of the experiments. Also, the controls applied for evaluation of the results were in line with those of EXAMPLE 1 (iPC, sPC, NC):

iPC: is the internal assay control where cells were treated throughout the experiment with cell culture medium and did not undergo dehydration

sPC: is the sample positive control where cells were treated with a mixture of one part sample medium and one part cell culture medium throughout the experiment and did not undergo dehydration

NC: is the negative control where cells were pre-treated solely with cell culture medium and subjected to drying conditions thereafter.

The following day the seeding medium was replaced with the samples referred to in Table 3 diluted 1:2 with CCM. For the iPC and NC the seeding medium was replaced with cell culture medium only, i.e. not containing any of the active ingredients. The cells were incubated for 2 h and thereafter subjected to dehydrating conditions following the protocol of Example 1. The cell viability was evaluated as described in EXAMPLE 1.

The bar chart of Figure 3 exhibits the results of cornea cell protection by the tested commercial products given as percentage of cell survival relative to the corresponding sPC, determined after dehydrating the sample preparations for 10 minutes under the conditions mentioned in Example 1 above.

Figure 4 and Table 4 exhibit a comparison of the cell protective efficacy of the individual commercial preparations, expressed as the average time during which 50% of the corneal cells were kept alive, i.e. metabolically active, after dehydrating treatment for up to 40 minutes.

Table 4: Half-lives (in ascending order) of corneal cells pre-treated with commercially available eye drop preparations and thereafter subjected to dehydrating conditions.

| Sample | Half-Life [min] | Lower Limit (CI 95%) | Upper Limit (CI95%) |
|---|---|---|---|
| NC | 3.58 | 2.88 | 4.28 |
| 7 /HD | 5.27 | 3.62 | 6.93 |
| 3 /TH | 5.52 | 4.14 | 6.90 |
| 8 /TD | 5.78 | 4.59 | 6.98 |
| 2 /HC | 6.10 | 4.40 | 7.80 |
| 6 /BA | 7.66 | 6.45 | 8.86 |
| 4 /VM | 8.40 | 7.08 | 9.73 |
| 5 /VE | 10.12 | 7.88 | 12.36 |
| 1 /EL | 15.09 | 11.87 | 18.31 |

The results of this comparative study revealed a remarkable superiority of the present iota-carrageenan preparation over all tested marketed eye-drop products. The iota-carrageenan based composition prepared in accordance with the present invention (Sample 1, EL) demonstrated consistent and effective moisturizing and cell protective properties, proving its excellent suitability as a medicament for protecting the ocular surface against dehydration.

Moreover, it can also be taken from the results disclosed in this example that low concentrations of hyaluronic acid, such as for example in Sample 7 (HD, 0.05% HA), as well as the use of low molecular weight hyaluronic acid, as for example in Sample 3 (TH, 360 kDa HA) yielded only poor cell protective results. Formulations with high molecular weight hyaluronic acid, as for example in Sample 2 (HC, 3.3 MDa HA), as well as formulations comprising medium molecular weight hyaluronic acid, as for example in Sample 6 (BA, 1.1 MDa HA), resulted in modest protective results. Whereas formulations comprising high concentrations of hyaluronic acid, such as for example Sample 5 (VE, 0.24% HA) performed better.

Consequently, the observed extraordinary superiority of iota-carrageenan with regard to moisturizing capacity and cell protective efficacy recommends its use as a main or the sole active ingredient in the manufacture of a medicament for the prophylactic or therapeutic treatment of dry eye symptoms and related conditions.

EXAMPLE 3: Test for mucoadhesive properties using viscosimetry

The key features of eye-drops include mucoadhesion and moisturization for providing moisture to the eye surface and for lubricating the cornea as well as the conjunctiva, thereby reducing the friction between the eyelids and the cornea and minimizing discomfort to the eye. A mucoadhesive polymer is characterized in that its dynamic viscosity in a mixture together with mucin is higher than the mere additive effect of the dynamic viscosities measured for each of the two components independently. This effect is due to an interaction between the polymer and mucin in an aqueous solution.

The viscosity of iota-carrageenan and hyaluronic acid is strongly influenced by $Na^+$ ions which are present in the mucin preparation due to the required buffer.

For the purpose of viscosity measurement, a 15% w/w mucin solution was prepared and mixed with a 0.1M $Na_3PO_4$ solution and the pH of the mixture adjusted to pH 7.04 using 1M NaOH solution. In addition to the present eye-drop preparation comprising 1.2 mg/l iota-carrageenan in 0.5% NaCl solution, three other commercial eye-drop preparations were enrolled with this test for comparative purposes, namely preparation SY (comprises paraffin and guarapolose as active ingredients), TD (see Table 3 above, contains hyaluronic acid in combination with trehalose), and HD (see Table 3 above, contains HA in combination with ectoin).

All mucin containing experimental samples were adjusted to contain comparable concentrations of phosphate and sodium ions in the experimental mixtures. The samples ready for viscosimetric measurement contained 5% mucin along with 67% of the original eye-drop solution. They were prepared and kept at 35°C for subsequent viscosimetric measurements at the same temperature. Each experimental sample was measured three times at increasing shear rates of from 2.4 to 44 or 64 [1/s] with a 2-minute break between each measurement cycle, using a rotational viscosimeter IKA, Rotavisc lo-vi S000, equipped with a co-axial cylinder system with water jacket IKA, ELVAS-1. In addition, respective control samples without mucin were also subjected to viscosimetric measurements using the same equipment and conditions.

The shear rate [1 /s] is a function of the spindle type and cup including their exact dimensions and the rotational speed [rpm]. The latter is a direct variable in the controls of the viscosimeter, but viscosity [mPa*s] is given as a function of shear rate. Measurement results are recorded automatically for every second of the time span of each shear rate. Larger time spans were chosen for the lower shear rates to measure an adequate number of rotations. The mucoadhesive strength was calculated using the following equation:

$$\Delta\,(\%) = [\eta muc + \text{test item} - (\eta muc + \eta \text{test item})]/(\eta muc + \eta \text{test item}) \times 100$$

expressing the increase or decrease of viscosity of the experimental mixtures of mucin and polymer in % of the calculated sum of independently determined viscosities of the mucin and polymer solutions.

Figure 6 displays the results of viscosity measurements of the iota-carrageenan preparation (2.13mg/ml), the mucin solution (5%), and of the mixture of iota-carrageenan plus mucin. Additionally, Fig. 6 also indicates the additive value of the independently determined viscosities of the iota-carrageen solution and the mucin solution, respectively.

Figure 7 compares the mucoadhesive strength of the tested eye-drop sample preparations comprising 5% mucin.

Figure 8 exhibits a comparison of mucoadhesive strength of sample preparations comprising a polymer + mucin. More specifically, the polymers are selected from iota-carrageenan (2.13 mg/ml) and commercial eye-drop preparations comprising hyaluronic acid as an active polymer ingredient. Mucoadhesion or mucoadhesive strength is expressed as percentage of viscosity difference ($\Delta\%$), i.e. viscosity increase, of the mucin containing sample preparations over the mere sum of viscosities independently determined for the respective mucin and polymer solutions. All measurements have been done at a shear rate of 3 [1 /s].

The comparison of the eye-drop samples with respect to their mucoadhesive properties confirms that the commercial eye-drop preparations SY, TD and HD when combined with mucin go with an increase in viscosity of between 15 and 27 % compared to the calculated sum of independently determined viscosities of the pure mucin and respective eye-drop preparations at a shear rate of 3 [1/s]. However, at the same shear rate the viscosity of mixture of mucin and iota-carrageenan exceeded the calculated additive value by more than 110 %.

Because of its shear thinning nature, iota-carrageenan's mucoadhesive properties did not remain constant over the range of shear rates investigated. Nevertheless, at a shear rate of 34.2 [1 /s] the increase over the calculated additive value is still 40 % and therefore superior to the one obtained with the comparative commercial eye-drop preparations SY, TD and HD, which remain at a Newtonian plateau throughout the entire test range.

It can be derived therefrom, that the present iota-carrageenan composition will spread quickly and reliably over the ocular surface under the shear stress of a blinking eye lid, which shear stress according to literature may reach up to 1000 [1/s]. Even more so, thanks to its excellent mucoadhesive properties iota-carrageenan adheres well to the ocular surface and surrounding mucosal tissues.

EXAMPLE 4: Barrier functionality of selected polymers

In this experiment, different polymers namely dextran sulfate, fucoidan from Undaria pinnatifida, fucoidan from Fucus vesiculosus, degraded iota-carrageenan (low molecular weight 100 - 500 kDa), CMC of high viscosity, high molecular weight iota-, kappa- and lambda-carrageenan (3-10 MDa), and HPMC, were tested and compared for their capability to inhibit the migration of particles through a barrier layer in an in vitro assay. More specifically, the assay was designed to investigate the effectiveness of a polymer, i.e. polysaccharide, to function as a barrier and prevent the passage of specific substances such as allergens, particulate matter including pollen, dust and fine dust particles, animal dander, and antibodies, from one side of the barrier layer to the other side. The results obtained allow for repeatable assessment of a given polymer's blocking capability towards specific substances.

Abbreviations used:

CMC  carboxymethyl cellulose
Der p1  house dust mite allergen
HPMC  hydroxypropylmethyl cellulose
PM  particulate matter
PBS  phosphate buffered saline
FITC  fluorescein isothiocyanate
Fab'  antigen binding antibody fragment
RT  room temperature
BC  blank control
NC  negative control
PC  positive control
CV  coefficient of variation
SD  standard deviation
UCL  upper control limit
LCL  lower control limit

For investigating possible differences among the selected polymers with respect to their abilities to inhibit the migration of particulate matter through a model barrier layer, a standardized barrier assay was used in which fluorescent beads of a size of 0.3 $\mu$m and 1.0 $\mu$m were applied as surrogate material for particulate matter in general. All polymers were dissolved in 0.5% NaCl at a final concentration of 1.2 mg/ml. The amounts of barrier-crossing beads were determined 180 minutes after application of the beads. The error bars represent the standard deviation of 3 replicates.

A composition comprising iota-carrageenan at a concentration of 1.2 mg/ml in a 0.5 % sodium chloride solution was used as a positive control (PC), and the same sodium chloride solution without iota-carrageenan served as a negative control (NC). Depending on the particulate material or agent to be tested, ELISA, qPCR, or other detection methods can be used instead of fluorometry for the quantification of particle migration.

Assay Procedure

- Agar plates were prepared at least one day before the assay started by filling each well of the 1.1 ml 96-deep well plate with 250 $\mu$l of a 1.25 % agar solution;
- the plates were stored overnight covered with an adhesive foil at 4 °C;
- each sample and control were analyzed in quadruplets

    PC: 1.2 mg/ml iota-carrageenan in 0.5% w/v NaCl solution
    NC: is a 0.5 % w/v NaCl solution
    BC: no solution is added on the agar block;

- 200 $\mu$l of the samples and of the controls were added to the agar surfaces leaving four wells empty for the blank control. The solutions were allowed to settle for 5 minutes at RT in order to build a barrier layer on top of the agar surfaces. Those wells left empty had been covered with an adhesive foil.
- for the positive and negative controls 10 $\mu$l of a solution comprising 10 $\mu$g/ml of a fluorescence-labeled antibody, i.e. FITC labeled anti-mouse IgG (Fab')2, were added resulting in a final amount of 100 ng of antibody;
- for determining the migration of particulate material through the experimental barrier layer the particulate material was added at a predetermined concentration in a volume of 10 $\mu$l;
- the plates were then incubated at RT over a preselected time period, e.g. 1 hour, specifically adapted to the particulate material tested;
- after incubation all wells were washed 5x with 500 $\mu$l washing buffer;
- 500 $\mu$l of an extraction buffer were added on the washed agar blocks and allowed for incubation over night at 4 °C on a shaker rotating at 900 rpm;
- the next day the supernatant was transferred into a 96-deep-well plate where the supernatants were gently mixed;
- before proceeding with the further sample preparation for the specific detection the assay controls were analyzed: 100 $\mu$l of the controls (blank-, positive-, negative-control) were transferred into a 96-well black flat bottom plate and analyzed using a plate photometer (e.g. Fluostar Omega microtiter plate reader, BMG Labtech) at an excitation and emission wavelength of 485 nm and 520 nm, respectively;
- if all control samples met the acceptance criteria defined for this assay, the supernatant of the test samples were further processed according to the detection method used to quantify the specific particulate material or agent, e.g., ELISA for allergens.

Samples and controls were analyzed in quadruplets. For safeguarding accuracy and correctness of the assay and its results, several controls were included in the assay procedure:

Positive Control (PC):
A commercially available iota-carrageenan preparation (1.2 mg/ml in 0.5% NaCl solution) having a known blocking activity was included in each assay to ensure that the assay is functional and allows for relating the blocking activity of a test polymer to the reference polymer having a known blocking activity.

Negative control (NC):
The negative control comprises the matrix of the positive control (0.5 % NaCl) without any blocking polymer. It represents the maximal diffusion capability of the FITC-labeled antibody and is set to 0 % blocking activity.

Blank control (BC):
As blank control the agar without added barrier polymer is extracted with 500 $\mu$l extraction buffer like the samples, and the supernatant is measured fluorometrically to analyze the fluorescence background of the lysis buffer / agar which will be subtracted from each sample.

Table 5: Barrier polymers applied in the blocking assay

| Polymers / Source | Supplier | Concentration |
|---|---|---|
| iota-carrageenan / Gelcarin PH379 | Du Pont Nutrition | 1.2 mg/ml in 0.5% NaCl |
| kappa-carrageenan / Gelcarin PH911 | Du Pont Nutrition | 1.2 mg/ml in 0.5% NaCl |

(continued)

| Polymers / Source | Supplier | Concentration |
|---|---|---|
| lambda-carrageenan / Viscarin PH109 | FMC | 1.2 mg/ml in 0.5% NaCl |
| dextran sulphate sodium salt, from Leuconostoc spp. average mol wt > 500,000 (dextran starting material), contains 0.5-2.0% phosphate buffer, pH 6-8 | Sigma | 1.2 mg/ml in 0.5% NaCl |
| fucoidan from Undaria pinnatifida, purity > = 95% | Sigma | 1.2 mg/ml in 0.5% NaCl |
| fucoidan from Fucus vesiculosus, purity > = 95% | Sigma | 1.2 mg/ml in 0.5% NaCl |
| CMC, high viscosity carboxymethyl cellulose | Hercules | 1.2 mg/ml in 0.5% NaCl |
| HPMC E4M PREM | Fagron | 1.2 mg/ml in 0.5% NaCl |

Table 6: Applied carrageenan material

| Substance and quality | Approx. Size [MDa] |
|---|---|
| iota-carrageenan (Gelcarin PH379) | 7 (5 - 9 ) |
| kappa-carrageenan (Gelcarin PH911) | 9 (7- 10) |
| lambda-carrageenan (Viscarin PH 109) | 9 (7-10) |
| degraded iota-carrageenan (1 h hydrolyzation) | 371 kDa |
| degraded iota-carrageenan (3h hydrolyzation) | 218 kDa |

The surprising results are represented in Figs. 5A, 5B and 5C. It can be taken from the figures that the blocking efficacy of the polymers varies tremendously and unexpectedly among the selected polymers, irrespective of the size or nature of the particulate material tested.

The size of the particulate matter tested seemed to only slightly, if at all, carry any effect on the blocking capacity of the polymers. As depicted in Figs. 5A and 5C, out of the nine polymers tested only four were able to block the migration of the test-beads. Iota-carrageenan was the most effective polymer preventing 92% of the 0.3 $\mu$m beads and 85% of the 1 $\mu$m beads from migration through the barrier layer, followed by lambda-carrageenan (79% and 67%), HPMC (73% and 53%) and dextran sulfate (39% and 25%).

However, it shall be mentioned that commercially available "lambda-carrageenan" contains prevailingly kappa- and iota-carrageenan fractions and only relatively low amounts of lambda-carrageenan. More precisely, the composition of the lambda-carrageenan material used in this study comprised, as determined by NMR detection, 41.8 %wt kappa-carrageenan, 27.3 %wt iota-carrageenan and only 19.6 %wt lambda-carrageenan. Therefore, the relatively high blocking efficacy of the lambda-carrageenan material may probably be attributable to the iota-carrageenan fraction present in that material.

The results also represent that low molecular weight iota-carrageenan (obtained through chemical degradation by treatment with 1N HCl for 1 hour or 3 hours at room temperature) was entirely ineffective as a barrier material. Most surprisingly, however, the remaining polymers including kappa-carrageenan, which is structurally very similar to iota- and lambda-carrageenan, were also completely ineffective with respect to blocking particle migration (Figs 5A, 5B).

In addition, as represented in Fig. 5C, it was demonstrated that iota-carrageenan is also 1.4 to 2.3-fold better in blocking the passage of the house dust mite allergen (Der p1) through the barrier layer than all other polymers tested, i.e. iota-carrageenan blocked the passage of the tested allergen by almost 90%, whereas the blocking efficacies of the other polymers ranged from 38% to 62%. The amounts of barrier-crossing material were analyzed 120 minutes after allergen application. The error bars represent the standard deviation of 4 replicates.

These unexpected results once again highlight the superiority of iota-carrageenan over some important polymers used in commerce and thus impressively confirm its potential to protect sensitive mucosa as well as the cornea and the surface of an individual's eye from drying out as well as from possible damage or contamination with particulate and/or allergenic matter typically conveyed through moving air and winds.

Accordingly, the pharmaceutical compositions prepared in accordance with the present invention typically containing iota-carrageenan as the main or sole active ingredient may be beneficially used as ophthalmic preparations for preventing or relieving symptoms due to non-viral conjunctivitis or dryness of the eye.

They contain iota-carrageenan at a concentration in a range of from 0.5 to 6 mg/ml, or from 1.0 to 4.5 mg/ml, or from 1.5 to 3.5 mg/ml. The ophthalmic preparations in line with the invention are preferably adapted as eye drops and are typically administered to an individual's eye from 1 to 10 times a day, usually 3-5 times a day, depending on the clinical status of the affected eye(s) and on the personal needs of the recipient.

Iota-carrageenan has excellent water uptake, water retention and mucoadhesive properties. It forms soft gels in contact with water and interacts with secreted mucins to build a stable mucous layer. In combination with its barrier functionality against particulate material and allergens the naturally occurring iota-carrageenan not only forms a protective barrier film on the ocular surface thereby protecting the eye against excessive evaporation of the liquid tear film and simultaneously lubricating the eye surface, it also supports the body's natural defense mechanisms against intrusion of airborne allergens, pollen and other particulate matter frequently compromising an individual's eye.

EXAMPLE 5: Clinical investigation of eye-protective efficacy in vivo.

The iota-carrageenan based ophthalmic composition administered to the recipients in the form of eye-drops was comprised of the following ingredients:

| | |
|---|---|
| 0.320 g | iota carrageenan; |
| 4.0 g | sorbitol |
| 0.100 g | sodium edetate (EDTA) |
| 0.367 g | disodiumdihydrogenphosphate dihydrate |
| 0.046 g | citric acid monohydrate |
| Purifiedwater to 100 ml | |

A clinical investigation to evaluate the lubricating and soothing performance of eye drops based on iota-carrageenan as the active ingredient was performed in patients diagnosed with mild-to-moderate dry eye disease (DED). The primary endpoint was a composite score on DED related ocular symptoms collected through numerical rating scales (NRS) for foreign body sensation, burning/ stinging, itching, pain, sticky feeling, blurred vision, and photophobia. Secondary endpoint patient responder rates were evaluated as per the NRS scores. The patients applied one drop of the preparation per eye three times a day for 28 days. The effectiveness of the eye drops was assessed 14 days and 28 days after the first treatment. The average baseline symptom score of 7 patients was 17.57 at baseline and decreased to 9.29 when measured in adverse controller environmental conditions (23°C, 10% relative humidity, airflow) and to 10.86 when measured in normal controlled environmental conditions (23°C, 50% relative humidity, no airflow). It can therefore be concluded that the treatment with iota-carrageenan eye-drops resulted in a clinically meaningful reduction of the NRS composite score on day 28 when compared to the baseline.

As a collateral revelation, it was surprisingly found that the intraocular pressure (IOP) of all treated patients was reduced by 20% already after 14 days of treatment when compared to the baseline. A similar reduced IOP level compared to the baseline was found when the patients returned for follow-up safety and health care checks after additional 14 days of treatment. The data suggest that the application of iota-carrageenan eye-drops might qualify as a novel non-invasive treatment technique suitable of supporting and broadening the therapeutic options available for the treatment of glaucoma.

Glaucoma is a leading cause of irreversible blindness across the globe. The primary goal of contemporary medical treatment of glaucoma is the reduction of intraocular pressure (IOP). However, medical management is often limited by poor adherence, polypharmacy, as well as local and systemic side effects. Extended-release drug delivery devices or entirely new therapeutical methods such as the one disclosed herein in line with the present invention could therefore substantially contribute to overcoming some of these limitations.

**Claims**

1.  Iota-carrageenan for use as an active ingredient in an aqueous pharmaceutical composition for the prevention or treatment of clinically relevant eye conditions affecting at least one of mucosal tissues at or around an individual's eye, an eye surface, and corneal tissue, said clinically relevant eye conditions selected from the group consisting of dry eye, non-viral conjunctivitis, irritations caused by airborne particulate matter or allergens, and increased intraocular pressure.

2.  Iota-carrageenan for the use claimed in claim 1, wherein said airborne particulate matter comprises dust particles, fine dust particles, pollen particles, animal dander, and airborne particles of leaves and of other vegetable material.

3. Iota-carrageenan for the use claimed in claim 1 or 2, wherein said use comprises administering an effective dose of the aqueous pharmaceutical composition to an individual's eye for wetting mucosal surfaces at and around the eye and for building an effective barrier layer on the eye's surface suitable to prevent the ocular surface and corneal tissue from intrusion and possible damage by airborne particulate matter and allergens.

4. Iota-carrageenan for the use claimed in claim 4, wherein said use comprises adhesion of the active ingredient to mucosal surfaces and corneal tissue and prevention of said mucosal surfaces and corneal tissue from drying out.

5. Iota-carrageenan for the use claimed in any one of claims 1 to 4, wherein the aqueous pharmaceutical composition in its ready-for-use formulation comprises iota-carrageenan as the main or sole active ingredient in an effective concentration of from 0.5 to 6 mg/ml, or from 1.0 to 4.5 mg/ml, or from 1.5 to 3.5 mg/ml.

6. Iota-carrageenan for the use claimed in any one of claims 1 to 5, wherein the pharmaceutical composition in its ready-for-use formulation contains no more than 0.5% w/v of a metal halide salt, said metal halide salt preferably selected from the group consisting of sodium chloride and potassium chloride.

7. Iota-carrageenan for the use claimed in any one of claims 1 to 6, wherein the pharmaceutical composition comprises at least one ophthalmologically compatible additive selected from the group consisting of a pH adjusting agent, a surfactant, a preservative, and an antioxidant.

8. An aqueous pharmaceutical composition comprising iota-carrageenan as the main or sole active ingredient, for use as a medicament in the prophylactic or therapeutic treatment of clinically relevant eye conditions affecting at least one of mucosal tissues at or around an individual's eye, an eye surface, and corneal tissue, said clinically relevant eye conditions selected from the group consisting of dry eye, non-viral conjunctivitis, irritations caused by airborne particulate matter or allergens, and increased intraocular pressure.

9. The aqueous pharmaceutical composition for the use claimed in claim 8, said use comprising administering an effective dose of the aqueous pharmaceutical composition to an individual's eye for wetting mucosal surfaces at and around the eye and for building an effective barrier layer on the eye's surface suitable to prevent the ocular surface and corneal tissue from intrusion and possible damage by airborne particulate matter and allergens.

10. The aqueous pharmaceutical composition for the use claimed in claim 8 or 9, wherein said use comprises adhesion of the active ingredient to mucosal surfaces and corneal tissue and prevention of said mucosal surfaces and corneal tissue from drying out.

11. The aqueous pharmaceutical composition for the use claimed in any one of claims 8 to 10, wherein the aqueous pharmaceutical composition in its ready-for-use formulation comprises iota-carrageenan as the main or sole active ingredient in an effective concentration of from 0.5 to 6 mg/ml, or from 1.0 to 4.5 mg/ml, or from 1.5 to 3.5 mg/ml.

12. The aqueous pharmaceutical composition for the use claimed in any one of claims 8 to 11, wherein the pharmaceutical composition in its ready-for-use formulation contains no more than 0.5% w/v of a metal halide salt, said metal halide salt preferably selected from the group consisting of sodium chloride and potassium chloride.

13. The aqueous pharmaceutical composition for the use claimed in any one of claims 8 to 12, wherein the pharmaceutical composition comprises at least one ophthalmologically compatible additive selected from the group consisting of a pH adjusting agent, a surfactant, a preservative, and an anti-oxidant.

14. Iota-carrageenan for use as an active ingredient in the manufacture of an aqueous pharmaceutical composition for the prevention or treatment of clinically relevant eye conditions affecting at least one of mucosal tissues at or around an individual's eye, an eye surface, and corneal tissue, said eye conditions selected from the group consisting of dry eye, non-viral conjunctivitis, and irritations caused by airborne particulate matter and allergens.

15. Iota-carrageenan for the use claimed in claim 14, wherein said prevention or treatment comprises administering an effective dose of the aqueous pharmaceutical composition to an individual's eye for wetting mucosal surfaces at and around the eye and for building an effective barrier layer on the eye's surface suitable to prevent the ocular surface and corneal tissue from intrusion and possible damage by airborne particulate matter and allergens.

FIG.1A

FIG.1B

FIG. 2A

FIG. 2B

FIG. 3

FIG. 4

FIG. 5A

FIG. 5B

FIG. 5C

FIG. 6

FIG. 7

FIG. 8

Europäisches
Patentamt

European
Patent Office

Office européen
des brevets

# EUROPEAN SEARCH REPORT

**Application Number**

EP 24 16 7732

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Marinomed: "Carragelose eye drops for lubrication and beyond", , 1 November 2023 (2023-11-01), XP093198535, Retrieved from the Internet: URL:https://www.marinomed.com/fileadmin/02 _Carragelose/03_Business_Development/Carra gelose_eyedrop_teaser_final_01.pdf * the whole document * | 1-15 | INV. A61K31/731 A61K9/00 A61P27/04 A61P27/06 A61P27/14 |
| X | US 2011/091583 A1 (GRASSAUER ANDREAS [AT] ET AL) 21 April 2011 (2011-04-21) * paragraph [0014]; claims 18,20,26,28; examples * | 1,8,14 | |
| A | US 2016/331776 A1 (PRIESCHL-GRASSAUER EVA [AT] ET AL) 17 November 2016 (2016-11-17) * paragraph [0009]; claims; examples * | 1-15 | |
| A | RAWAT PRADEEP SINGH ET AL: "Design, Characterization and Pharmacokinetic-Pharmacodynamic Evaluation of Poloxamer and Kappa-Carrageenan-Based Dual-Responsive In Situ Gel of Nebivolol for Treatment of Open-Angle Glaucoma", PHARMACEUTICS, vol. 15, no. 2, 25 January 2023 (2023-01-25), page 405, XP093198516, Switzerland ISSN: 1999-4923, DOI: 10.3390/pharmaceutics15020405 * the whole document * | 1-15 | **TECHNICAL FIELDS SEARCHED (IPC)** A61K A61P |

The present search report has been drawn up for all claims

| Place of search | Date of completion of the search | Examiner |
|---|---|---|
| Munich | 30 August 2024 | Moutafidou, Nikoleta |

EPO FORM 1503 03.82 (P04C01)

## ANNEX TO THE EUROPEAN SEARCH REPORT
## ON EUROPEAN PATENT APPLICATION NO.

EP 24 16 7732

This annex lists the patent family members relating to the patent documents cited in the above-mentioned European search report.
The members are as contained in the European Patent Office EDP file on
The European Patent Office is in no way liable for these particulars which are merely given for the purpose of information.

30-08-2024

| Patent document cited in search report | Publication date | Patent family member(s) | | Publication date |
|---|---|---|---|---|
| US 2011091583 A1 | 21-04-2011 | AU | 2009266076 A1 | 07-01-2010 |
| | | BR | PI0915119 A2 | 10-02-2016 |
| | | CA | 2726221 A1 | 07-01-2010 |
| | | CN | 102065869 A | 18-05-2011 |
| | | DK | 2303287 T3 | 17-02-2014 |
| | | EA | 201170117 A1 | 30-06-2011 |
| | | EP | 2303287 A1 | 06-04-2011 |
| | | ES | 2445870 T3 | 05-03-2014 |
| | | HR | P20140164 T1 | 11-04-2014 |
| | | JP | 5687619 B2 | 18-03-2015 |
| | | JP | 2012531382 A | 10-12-2012 |
| | | NZ | 589293 A | 28-09-2012 |
| | | PL | 2303287 T3 | 30-05-2014 |
| | | PT | 2303287 E | 27-02-2014 |
| | | SG | 191697 A1 | 31-07-2013 |
| | | SI | 2303287 T1 | 30-04-2014 |
| | | US | 2011091583 A1 | 21-04-2011 |
| | | US | 2012058206 A1 | 08-03-2012 |
| | | WO | 2010000437 A1 | 07-01-2010 |
| US 2016331776 A1 | 17-11-2016 | CA | 2937402 A1 | 30-07-2015 |
| | | CN | 105939720 A | 14-09-2016 |
| | | DK | 2898888 T3 | 03-06-2019 |
| | | EP | 2898888 A1 | 29-07-2015 |
| | | EP | 3096766 A1 | 30-11-2016 |
| | | ES | 2734269 T3 | 05-12-2019 |
| | | ES | 2791360 T3 | 04-11-2020 |
| | | HR | P20191050 T1 | 06-09-2019 |
| | | HU | E044329 T2 | 28-10-2019 |
| | | JP | 6635343 B2 | 22-01-2020 |
| | | JP | 2017503841 A | 02-02-2017 |
| | | PL | 2898888 T3 | 30-09-2019 |
| | | PT | 2898888 T | 16-07-2019 |
| | | SI | 2898888 T1 | 28-06-2019 |
| | | US | 2016331776 A1 | 17-11-2016 |
| | | WO | 2015110429 A1 | 30-07-2015 |
| | | WO | 2015110430 A1 | 30-07-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5403841 A
- WO 2007039201 A
- WO 2005046562 A